# EUROPÄISCHE PATENTSCHRIFT

(11) **EP 2 557 254 B1**
(45) Veröffentlichungstag und Bekanntmachung des Hinweises auf die Patenterteilung: **22.07.2015**
(21) Anmeldenummer: 11191812.4
(22) Anmeldetag: 02.12.2011
(51) Int. Cl.: E04H 1/12

(54) **Bernsteinraum zur Darstellung von Lichtbildern im Innenbereich des Raumes**
Amber room for displaying photos inside the room
Pièce d'ambre jaune pour la représentation d'images lumineuses dans l'espace intérieur de la pièce

(30) Priorität: 09.08.2011 DE 102011052537
(43) Veröffentlichungstag der Anmeldung: 13.02.2013
(73) Patentinhaber: Ungefucht, Robert, 92224 Amberg (DE)
(72) Erfinder: Ungefucht, Robert, 92224 Amberg (DE)
(74) Vertreter: Bittner, Bernhard

(56) Entgegenhaltungen:
- WO-A1-2010/110633
- US-A- 4 987 706

## Beschreibung

Die vorliegende Erfindung bezieht sich auf einen von der Außenumgebung abgegrenzten Raum zur Darstellung von Lichtbildern im Innenbereich des Raumes gemäß dem Oberbegriff des Patentanspruches 1.

Besonders im Wellness- und Fitnessbereich ist die Verwendung von Ruhe- und Entspannungsräumen hinreichend bekannt, so dass diese beispielsweise zum Entspannen der Saunagäste nach einem Saunagang dienen und direkt an einem Saunabereich angrenzen.

Derartige Räume zeichnen sich besonders durch eine auf das Nervensystem der Person beruhigend wirkende Umgebung, welche beispielsweise mit entsprechenden Farbtönen oder einer minimalistischen Einrichtung versucht wird zu realisieren, aus, damit die den Raum besuchenden Personen vorzugsweise Erholung und Entspannung erfahren. Die Lichtquellen derartiger Räume sind zumeist gedimmt oder nur sporadisch gesetzt, um ein angenehmes warmes Licht im Raum zu erzeugen.

Jedoch sind diese aus dem allgemeinen Stand der Technik bekannten Räume zumeist nicht derart von der Außenumgebung abgegrenzt, dass die in dem Raum befindliche Person völlige Ruhe und Erholung finden kann. Zudem weißen die oft in den Räumen verwendeten Materialien gesundheitsschädliche Lacke und/oder Tenside auf, welche die in dem Raum befindliche Person beispielsweise über die Lungen aufnehmen könnte.

Oftmals fällt es auch bestimmten Personen sehr schwer sich in einem sehr ruhigen Raum völlig zu entspannen, so dass dieser Personenkreis eine Art aktiver Erholung benötigt. D.h., dass die Sinne dieser den Raum nutzenden Person derart von der Außenumgebung und seinen eigenen Gedanken und Problemen abgelenkt werden soll, so dass diese Person eine tiefe Entspannung und Ruhe erfahren kann.

Demnach ist es die Aufgabe der vorliegenden Erfindung einen von der Außenumgebung abgegrenzten und Im Wesentlichen schall- und lichtundurchlässigen Raum zur Darstellung von Lichtbildern in einem Innenbereich des Raumes zur Verfügung zu stellen, welcher mit vorwiegend natürlichen Rohstoffen bzw. Materialien aufgebaut wurde und vorzugsweise über farbliche Symbolen bzw. Bildern bzw. Lichtbildern den die Raum nutzende Person bzw. den Anwender bei seiner Entspannung unterstützt.

Diese Aufgabe löst die vorliegende Erfindung mittels eines Raumes gemäß dem Patenanspruch 1.

Dementsprechend wird ein von einer Außenumgebung abgegrenzter und im Wesentlichen schall- und lichtundurchlässiger Raum zur Darstellung von Lichtbildern in einem Innenbereich des Raumes mit mindestens einem Wandbereich aus mindestens vier Wänden, einem Bodenbereich, einem Deckenbereich und einem im Wesentlichen schall- und lichtundurchlässig verschließbaren Eingangsbereich sowie mit einer Holzkonstruktion zur Stabilisierung mindestens des Wandbereiches und des Bodenbereiches des Raumes beansprucht.

Dabei weist die Holzkonstruktion im Bodenbereich mindestens eine erste Holzplatte und eine darüber beabstandet angeordnete Bodenplatte auf, wobei zwischen der Bodenplatte und der ersten Holzplatte eine Vielzahl von Bernsteinen angeordnet sind.

Dokument WO 2010 11 06 33 offenbart einen Raum mit den Merkmalen des Oberbegriffs des Anspruchs 1.

Bernsteine sind ein natürliches Produkt, welches in gewissen Kreisen der Bevölkerung sogar den Ruf genießt eine beruhigende Wirkung zu besitzen und bei Allergien und Ekzemen eine wohltuende Wirkung zu entfalten.

Für die Holzkonstruktion und/oder die Holzplatte und/oder die Bodenplatte selbst wird bevorzugt Kiefernholz verwendet, welches, wie auch der Bernstein, einen natürlichen Rohstoff darstellt und sich zudem beispielsweise durch seine hohe Festigkeit auszeichnet. Anstatt des Kiefernholzes können jedoch auch andere Holzarten, wie beispielsweise Buchenholz und/oder Zedernholz usw., wie auch Kombinationen aus verschiedenen Holzarten verwendet werden.

Infolgedessen ist es möglich, dass die verwendete erste Holzplatte im Bodenbereich eine Kiefersperrholzplatte ist, welche vorzugsweise eine Dicke von 6mm aufweist. Es ist zudem denkbar, dass eine Vielzahl von Kiefernsperrholzplatten verwendet werden, welche nebeneinander angeordnet auf einem Holzgestell vorzugsweise verschraubt sind.

Das Holzgestell beabstandet dementsprechend den Boden, d.h. den Erdboden, wie beispielsweise die Wiese etc., je nachdem an welchem Ort der erfindungsgemäße Raum aufgestellt wird, von der ersten Holzplatte bzw. den ersten Holzplatten, so dass diese nicht direkt auf dem Erdboden aufliegen. Anstatt des Holzgestelles kann jedoch auch ein gegossener Betonboden bzw. ein Betongestell oder ein Eisengestell oder ein anderes tragendes Gestell oder eine weitere Bodenplatte verwendet werden, auf welchem die erste Holzplatte aufliegt.

Die erste Holzplatte und die Bodenplatte, welche ein zu der Holzplatte gleiches oder auch unterschiedliches Material aufweisen kann, sind vorzugsweise über eine Holzkonstruktion voneinander beabstandet. Demnach sind beispielsweise Holzbalken als Beabstandungsmittel zwischen der ersten Holzplatte und der Bodenplatte angeordnet, so dass sich im Bodenbereich ein Hohlraum zwischen der ersten Holzplatte und der Bodenplatte bildet.

Dieser Hohlraum wird vorzugsweise mit einer Vielzahl von Bernsteinen ausgefüllt.

Der Wandbereich des Raumes ist vorzugsweise ebenfalls durch die Holzkonstruktion gestützt bzw. stabilisiert, so dass diese Holzkonstruktion im Wandbereich mindestens eine zweite Holzplatte umfasst, welche durch eine Vielzahl von in der zweiten Holzplatte angeordneten Bohrungen gebildete Symbole bzw. Bilder bzw. Lichtbilder aufweist.

Demzufolge durchdringen die Bohrungen die zweite Holzplatte, die eine gleiche oder auch unterschiedliche Materialzusammensetzung als die erste Holzplatte und/oder Bodenplatte aufweisen kann, vorzugsweise vollständig und sind derart aneinander angeordnet, dass die Gesamtheit der Bohrungen bei einer Betrachtung im Ganzen ein Symbol bzw. ein Bild darstellen. Demnach bilden die Bohrungen die Linien des Bildes und weisen bevorzugt einen Durchmesser von 1 mm auf. Es ist jedoch auch denkbar, dass die Bohrungen jeweils einen unterschiedlichen Durchmesser aufweisen, so dass beispielsweise Bohrungen von 1 - 5mm vorliegen können, um durch derartige unterschiedliche Durchmesser auch eine unterschiedliche Lichtdurchdringung aufgrund beispielsweise unterschiedlich verwendeter Lichtleiter zu gewährleisten, wodurch einige Bereiche des Lichtbildes zum einen intensiver beleuchtet werden können, als andere Bereiche.

In einer bevorzugten Ausführungsform sind demzufolge durch die Bohrungen Lichtleiter in den Innenbereich des Raumes geführt, welche vor den Bohrungen angeordnete und im Innenbereich des Raumes befindliche Bernsteine beleuchten.

Diese Lichtleiter, welche vorzugsweise Glasfasern mit unterschiedlich einstellbarer Farbgebung sind, weisen bevorzugt einen Durchmesser von 0,75mm auf, können jedoch je nach Bohrung in der Holzplatte des Wandbereiches auch einen kleineren und/oder größeren Durchmesser als 0,75mm aufweisen.

Mittels beispielsweise einer Funkfernbedienung ist die Farbstrahlung der Lichtleiter einstellbar, wobei auch ein Wechselspiel der Farben der Lichtleiter eingestellt werden kann, so dass beispielsweise alle Lichtleiter eine zueinander identische oder auch unterschiedlich farbige Strahlung ermöglichen.

Demnach können beispielsweise die einzelnen Symbole in sich in unterschiedlichen Farben erstrahlen und/oder jedes einzelne Symbol bzw. Bild eine zu einem anderen Symbol unterschiedliche Farbe aufweisen.

In einer bevorzugten Ausführungsform weist die Bodenplatte eine Vielzahl von Luftlöchern auf, um beispielsweise eine Belüftung des Bernsteines zu ermöglichen. Durch diese Luftlöcher können jedoch auch ähnlich wie in den Wandbereichen bzw. Wänden des Raumes Lichtleiter hindurchgeführt und mit beispielsweise vor den Luftlöchern angeordneten Bernsteinen verbunden sein, durch welche diese Bernsteine beleuchtet werden.

Somit ist es möglich, dass die Luftlöcher entweder auch in Form eines Symbol im Bodenbereich bzw. der Bodenplatte angeordnet sind oder in einem im Wesentlich regelmäßigen Abstand zueinander angeordnet sind, so dass diese bei einer Beleuchtung beispielsweise, wie eine Art Sternenhimmel am Boden strahlen.

Demzufolge ist eine beliebige Anordnung der Luftlöcher möglich, je nachdem welchen Zweck diese erfüllen sollen, d.h. ob diese lediglich zur Belüftung des zwischen der Bodenplatte und der Holzplatte liegenden Bernsteins dienen oder auch eine definierte Beleuchtung des Bodens bzw. des Bodenbereiches realisieren sollen.

Bevorzugt weisen die Luftlöcher einen Durchmesser von 1 - 20mm und besonders bevorzugt von 5 - 10mm auf, wobei auch jeder andere Durchmesser gewählt werden kann. Zudem ist es denkbar, dass die in der Bodenplatte angeordneten Luftlöcher einen zueinander unterschiedlichen Durchmesser aufweisen können, so dass beispielsweise Luftlöcher mit einem größeren Durchmesser zur Belüftung des zwischen der Bodenplatte und der Holzplatte angeordneten Bernsteins dienen und Luftlöcher mit einem kleineren Durchmesser zur Beleuchtung des Bodenbereiches mittels durch die Löcher hindurch erstreckender Lichtleitern dienen. Infolgedessen muss nicht jedes Luftloch einen Lichtleiter aufweisen, welcher sich durch dieses Luftloch hindurch erstreckt.

In einer weiteren bevorzugten Ausführungsform sind die Luftlöcher über die Bodenplatte verteilt voneinander beabstandet und der Abstand der Luftlöcher zueinander beträgt vorzugsweise 40 - 80mm und besonders bevorzugt 50 - 60mm. Es ist jedoch, wie oben bereits erwähnt, denkbar, dass der Abstand der Luftlöcher, je nach Wunsch der Gestaltung des Bodenbereiches, unterschiedlich sein kann, so dass es möglich ist, dass jedes Luftloch zu einem weiteren Luftloch einen anderen Abstand aufweist. So wird eine unterschiedliche Bodengestaltung des Raumes ermöglicht, wobei gelichzeitig der zwischen der Bodenplatte und der ersten Holzplatte angeordnete Bernstein ausreichend belüftet werden kann.

Um eine hinreichende Belüftung des Innenbereiches des Raumes gewährleisten zu können, ist in mindestens einer Wand des Raumes und vorzugsweise in einer zweiten Holzplatte im Wandbereich mindestens eine Belüftungsbohrung angeordnet, welche Luft der Außenumgebung in den Innenbereich des Raumes leitet.

Dementsprechend durchbricht diese Belüftungsbohrung die zweite Holzplatte bevorzugt vollständig und stellt eine Verbindungsleitung zwischen dem Innenbereich des Raumes und der Außenumgebung dar.

In einer bevorzugten Ausführungsform ist innerhalb der Belüftungsbohrung mindestens ein erster Ventilator angeordnet, welcher Luft von der Außenumgebung in den Innenbereich des Raumes fördert. Dabei ist es denkbar, dass beispielsweise ein Axial- oder auch ein Radialventilator zum Einsatz kommen kann. Weiterhin ist es möglich, dass anstatt nur eines Ventilators auch zwei oder mehrere Ventilator nacheinander geschaltet vorzugsweise innerhalb der Belüftungsbohrung bzw. des Belüftungsschachtes angeordnet sind, oder dass ein auf der Außenseite des Raumes angeordneter Außenventilator und/oder ein in der Belüftungsbohrung angeordneter Ventilator angebracht sind. Der Anordnung und der Anzahl der Ventilatoren soll hierbei keine Grenze gesetzt werden.

Vorzugsweise ist eine Entlüftungsbohrung in dem Deckenbereich des Raumes angeordnet, um Luft aus dem Innenbereich des Raumes nach außen zu leiten. Dadurch soll es ermöglicht werden die von der in dem Raum befindlichen Person ausgeatmete Luft, welche kohlendioxidhaltig ist, aus dem Raum heraus zu transportieren, um der Person bzw. dem Verwender des Raumes vorzugsweise stetig frische sauerstoffreiche Luft zuführen zu können.

Die Zufuhr von frischer sauerstoffhaltiger Luft kann entweder, wie oben beschrieben, über die Belüftungsbohrung(en) ermöglicht werden, oder ein alternativer oder zusätzlicher, vorzugsweise auf der Außenseite des Raumes angeordneter Sauerstofftank ist über eine entsprechende Leitung mit dem Innenbereich des Raumes verbunden, um diesem Sauerstoff vorzugsweise in hochkonzentrierter Form zuzuführen.

Um den Abtransport der verbrauchten, d.h. der von dem Verwender ausgeatmeten Luft zu beschleunigen ist beispielsweise im Innenbereich des Raumes im Wesentlichen unterhalb der im Deckenbereich angeordneten Entlüftungsbohrung ein zweiter Ventilator angeordnet, welcher Luft im Innenbereich des Raumes umwälzt und diese Luft von dem Innenbereich des Raumes nach außen fördert.

Dieser Ventilator ist vorzugsweise ein handelsüblicher Deckenventilator, kann jedoch auch ein Radial- oder Axialventilator sein, welcher auch innerhalb der Entlüftungsbohrung angeordnet ist. Zudem ist es auch hier denkbar, dass beispielsweise zwei oder eine Vielzahl von Ventilatoren nacheinander angeordnet sind, so dass beispielsweise ein Deckenventilator im Wesentlichen vor der Entlüftungsbohrung und ein zweiter und eventuell auch dritter Ventilator innerhalb der Entlüftungsbohrung angeordnet sind.

Zur Ermöglichung einer vorzugsweise angenehmen Klimatisierung des Innenbereiches des Raumes ist vorzugsweise an dem Deckenbereich des Raumes eine Wärmequelle angeordnet, um den Innenbereich des Raumes zu erwärmen.

Diese Wärmequelle kann beispielsweise ein Infrarot-Strahler sein, welcher durch die Abgabe von Wärmestrahlung den Innenbereich des Raumes und vorzugsweise die in dem Raum befindliche Person erwärmt.

Des Weiteren weist eine bevorzugte Ausführungsform mindestens einen Zerstäuber auf, welcher vorzugsweise im Deckenbereich des Raumes angeordnet ist, jedoch auch im Bodenbereich und/oder an den Wänden des Raumes angeordnet sein kann und feinsten Bernsteinstaub in das Innere des Raumes einbläst bzw. in diesem Raum zerstäubt. Dadurch wird vorzugsweise ein von einer den Raum nutzenden Person als harmonisch empfundener Duft im Raum verbreitet, wodurch heilsame Wohlfühlatmosphäre geschaffen wird.

Es ist zudem möglich, dass auch im Deckenbereich bzw. in der Decke des Deckenbereiches, welche vorzugsweise auch aus einem zementartigen Material besteht, Bohrungen enthalten sind, welche sich derart durch den Deckenbereich bzw. die Deckenplatte bzw. die Decke erstrecken, dass sich auch durch diese Bohrungen Lichtleiter hindurch erstrecken können, welche dementsprechend vor den Lichtleitern angeordnete Bernsteine beleuchten bzw. anstrahlen. Durch eine, wie oben bereits aufgeführt, unterschiedliche Anordnung der Bohrungen zueinander können folglich entweder Symbole bzw. Bilder bzw. Lichtbilder erschaffen werden, oder die Decke kann in einer Art Sternenhimmel erstrahlen.

Vorzugsweise ist der Deckenbereich, wie auch die Wände bzw. der Wandbereich des Innenbereiches des Raumes vollständig mit Bernstein bedeckt.

Der Bodenbereich bzw. die Bodenplatte des Bodens kann ebenfalls vollständig mit Bernstein bedeckt werden. Es ist jedoch auch denkbar, dass oberhalb der Bodenplatte ein begehbarer Bodenbelag aufgebracht ist, welcher zu der Bodenplatte identische Luftlöcher aufweist. Demzufolge kann oberhalb der Bodenplatte beispielsweise Parkett, welches auch aus einem natürlichen Rochstoff hergestellt ist, angeordnet sein.

Vorzugsweise ist jedoch der Bodenbereich, wie auch der Wandbereich und/oder der Deckenbereich im Innenbereich des Raumes vorzugsweise vollständig oder auch teilweise mit Bernstein bedeckt.

Innerhalb des Raumes und bevorzugt im Deckenbereich des Raumes kann beispielsweise noch ein Rauchmelder angeordnet sein, um die Sicherheit der den Raum verwendenden Person zu gewährleisten, um einen möglicherweise durch die Lichtleiter und/oder die Wärmequelle und/oder die Ventilatoren und/oder dem Zerstäuber entstehenden Rauch zu melden und die Person dadurch zu warnen, so dass diese die Möglichkeit hat frühzeitig den Raum zu verlassen, ohne Schädigungen einer Rauchverletzung zu erhalten.

Des Weiteren ist es denkbar, dass der erfindungsgemäße Raum über eine Musikanlage mit entsprechend daran angeordneten Lautsprechern verfügt, wobei die Lautsprecher mit oder ohne die Musikanlage im Innenbereich des Raumes angeordnet sein können. D.h., die Musikanlage selbst kann außerhalb des Innenbereiches des Raumes, in welchem sich die Person aufhält, angebracht sein und lediglich ein entsprechendes Signal entweder über in den Raum gelegte Leitungen oder kabellos, beispielsweise über Funk, an die vorzugsweise im Innenbereich und bevorzugt im Deckenbereich des Innenbereiches des Raumes angeordneten Lautsprechern senden.

Es ist zudem denkbar, dass die einzelnen elektrischen bzw. elektronischen Geräte, wie z.B. die Ventilatoren und die Wärmequelle bzw. der Wärmestrahler bzw. die Wärmevorrichtung und der Zerstäuber beispielsweise derart über eine Steuereinrichtung, z.B. eines Computers oder vergleichbaren Maschinen, miteinander verbunden sind, dass die klimatischen Bedingungen im Innenbereich des Raumes z.B. über eine definierte Messeinrichtung ermittelt werden, wodurch die Wärmequelle und/oder die Ventilatoren gesteuert werden.

D.h., dass beispielsweise die Intensität der abgegebenen Wärmestrahlung der Wärmequelle reduziert wird, wenn die Messeinrichtung einen Temperaturwert ermittelt, welcher über einem definierten Temperaturwert liegt. Zugleich oder alternativ dazu kann die Drehzahl der Ventilatoren erhöht werden, um folglich ein Absinken der Temperatur im Innenbereich des Raumes zu realisieren.

Zudem ist es auch möglich über eine Messeinrichtung alternativ oder zusätzlich den Kohlendioxidgehalt in der Luft des Innenbereiches des Raumes zu messen, um beispielsweise bei einem Überschreiten eines definierten Kohlendioxid-Wertes den ersten und/oder den zweiten Ventilator derart anzusteuern, dass dadurch vermehrt frische Luft in den Innenbereich des Raumes geleitet und/oder vermehrt Luft aus dem Innenbereich des Raumes nach außen transportiert wird.

Diese Abstimmung der klimatischen Bedingungen sowie des Luft- bzw. Sauerstoffverhältnisses im Innenbereich des Raumes kann eine Steuereinheit bzw. eine Steuereinrichtung beispielsweise automatisch nach zuvor definierten Regelungswerten regeln bzw. steuern. Es ist jedoch auch denkbar, dass der Verwender des Raumes einzelne Geräte manuell ansteuert bzw. einstellt, beispielsweise mittels einer Funkfernbedienung, um die für sich am geeignetsten klimatischen Bedingungen zu ermöglichen.

Vorzugsweise wird immer eine identisch große Menge an Bernsteinstaub vom Zerstäuber in dem Raum eingebracht und zerstäubt. Mit Hilfe einer entsprechenden Messvorrichtung kann beispielsweise die Konzentration der eingebrachten Bernsteinstaubmenge im Raum gemessen und gegebenenfalls die vom Zerstäuber eingebrachte Bernsteinstaubmenge reguliert werden. Es ist jedoch auch denkbar, dass ein entsprechender Sensor bzw. ein entsprechendes Messgerät direkt am Zerstäuberausgang, aus welchen die Bernsteinstaubmenge in den Raum eingebracht wird, angeordnet ist, um eine Messung des durch den Zerstäuber in den Raum eingebrachten Bernsteinstaubes direkt d.h. unmittelbar messen zu können.

Weitere Vorteile, Ziele und Eigenschaften der vorliegenden Erfindung werden anhand nachfolgender Beschreibung anliegender Zeichnung erläutert, in welcher beispielhaft eine Prinzipskizze einer Ausführungsform des erfindungsgemäßen Raumes sowie des Bodenbereiches dargestellt wird.

Komponenten, welche in den Figuren wenigstens im Wesentlichen hinsichtlich ihrer Funktion übereinstimmen, können hierbei mit gleichen Bezugszeichen gekennzeichnet sein, wobei diese Komponenten nicht in allen Figuren gekennzeichnet und erläutert sein müssen.

In den Figuren zeigen:
- Fig.1: eine Prinzipskizze eines Längsschnittes durch eine Ausführungsform eines erfindungsgemäßen Raumes;
- Fig.2: eine Prinzipskizze eines Längsschnittes durch einen Bodenbereich einer Ausführungsform des erfindungsgemäßen Raumes; und
- Fig.3: eine Prinzipskizze eines Boden- und/oder Wand- und oder Deckenbereiches mit ein Symbol bildenden Bohrungen.

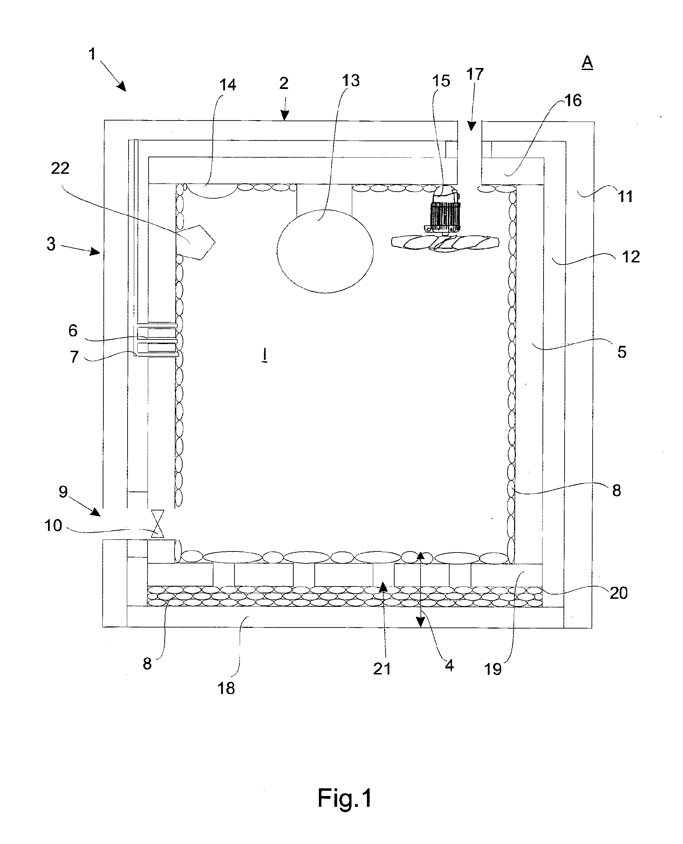

Fig.1 zeigt eine Prinzipskizze eines Längsschnittes durch eine Ausführungsform eines erfindungsgemäßen Raumes 1, welcher einen Deckenbereich 2, einen Wandbereich 3 mit mindestens vier Wänden und einen Bodenbereich 4 aufweist.

In mindestens einer Wand bzw. einer zweiten Holzplatte 5 des Wandbereiches ist eine Vielzahl von Bohrungen 6 eingebracht, welche sich vorzugsweise durch die gesamte Dicke der zweiten Holzplatte 5 erstrecken.

Diese Bohrungen können einen gleichen oder auch zueinander unterschiedlichen Durchmesser aufweisen und sind derart über den Wandbereich 3 bzw. die zweite Holzplatte 5 verteilt, dass diese gemeinsam betrachtet ein Symbol bzw. eine Bild erzeugen.
Durch diese Bohrungen 6 sind Lichtleiter 7 gesteckt, welche sich mit den vor den Bohrungen 6 im Innenbereich I des Raumes 1 an der zweiten Holzplatte 5 angeordneten Bernsteinen 8 verbinden, bzw. bis in diese hineinragen und diese Bernsteine 8 folglich beleuchten.

Demnach erstrahlen die Bernsteine 8 im Licht der Lichtleiter 7, deren Strahlung vorzugsweise je nach Kundenbedürfnis in der Farbe und/oder Helligkeit bzw. Intensität gewechselt werden kann.

Des Weiteren weist zumindest eine Wandung des Wandbereiches 3 zumindest eine Belüftungsbohrung 9 auf, welche sich vorzugsweise durch die gesamte Wandstärke des Wandungsbereiches 3 erstreckt und somit einen Schacht ausbildet, durch welchen es ermöglicht wird Frischluft von der Außenumgebung A, d.h. der Umgebung, welche den Raum 1 umgibt, in den Innenbereich I des Raumes 1 zu transportieren bzw. zu leiten.

Dieser Frischlufttransport wird unterstützt durch einen ersten Ventilator 10, welcher vorzugsweise in der Belüftungsbohrung 9 bzw. dem Belüftungsschacht 9 angeordnet ist, wobei der erste Ventilator 10 auch im Innenbereich I oder im Außenbereich A an der Wandung des Wandungsbereiches 3, jedoch dann vorzugsweise direkt vor dem Ein- bzw. Ausgang der Belüftungsbohrung 9 angeordnet sein kann.

Der Raum 1 wird vorzugsweise über eine Eingangstür (hier nicht gezeigt) betreten, welche in einer Wandung bzw. in einem Wandbereich 3 angeordnet ist und vorzugsweise eine schwingbare Tür mit einer Türklinke darstellt, welche über vorzugsweise an den Türkanten oder am Zargenrahmen befestigten Dichtungsprofilen derart licht- und/oder schalldicht verschlossen werden kann, das bei einer geschlossenen Tür weder Geräusche noch Licht bzw. Strahlung von der Außenumgebung A in den Innebereich I des Raumes 1 dringen kann.

Es ist jedoch auch denkbar, dass beispielsweise eine Schiebetür oder Falttür verwendet wird.

Die Tür ist vorzugsweise ebenfalls aus einem natürlichen Material bzw. Rohstoff, wie Holz gefertigt und weist vorzugsweise ebenfalls auf der zum Innenbereich I der Raumes 1 gerichteten Wandung Bernsteine auf, welche zudem, wie auch im Wandungs- und/oder Boden- und/oder Deckenbereich mittel Lichtleiter angestrahlt werden können. Derartige Lichtleiter sind dann vergleichbar zu den anderen Lichtleitern 7 im Raum 1 ebenfalls durch sich durch die Tür erstreckende Bohrungen geführt. Vorzugsweise ist die Tür doppelwandig und weist einen zwischen der Innen- und Außenwand der Tür bestehenden Hohlraum auf, in welchem die Kabel der Lichtleiter bzw. die Lichtleiter selbst angeordnet werden.

Der Deckenbereich 2 und/oder der Wandungsbereich 3 des Raumes 1 ist beispielsweise noch durch eine Außenwand 11, welche z.B. aus Beton besteht, umgeben, um die Holzplatten 5 von der Außenumgebung A abzugrenzen und einen Hohlraum 12 auszubilden, welcher beispielsweise verwendet wird, um die Kabel der Lichtleiter 7 und/oder der in dem Innenbereich I des Raumes 1 angeordneten Geräte, wie beispielsweise der Wärmequelle 13, dem Rauchmelder 14 und/oder dem ersten 10 und/oder zweiten Ventilator 15 verlegen zu können.

Der Rauchmelder 14 und die Wärmequelle 13 sind vorzugsweise an der Decke 16 des Deckenbereiches 2 angeordnet, welche ebenfalls aus einer Holzplatte oder auch vorzugsweise aus Beton gebildet ist.

Ein Zerstäuber 22 ist gemäß der Fig.1 an einem Wandungsbereich 3 des Raumes 1 angeordnet, kann aber auch, ähnlich wie beispielsweise der Rauchmelder im Deckenbereich 2 oder gar im Bodenbereich 4 des Raumes 1 angeordnet sein. Zudem ist es ebenso möglich, dass mehr als ein Zerstäuber 22 im Raum 1 angeordnet sind, beispielsweise zwei, drei oder mehr Zerstäuber 22. Diese Zerstäuber 22 verteilen sich dann beispielsweise über den Decken- 2, Wandungs- 4 und Bodenbereich 4 des Raumes 1, vorzugsweise im Wesentlichen gleichmäßig voneinander beabstandet, um eine im Wesentlichen gleichmäßige Zerstäubung des Bernsteinstaubes ermöglichen zu können.

Ein zweiter Ventilator 15 ist ebenfalls vorzugsweise an der Decke 15 des Deckenbereiches 2 angeordnet, um Luft im Innenbereich I des Raumes 1 zum einen aufzuwirbeln bzw. in Bewegung zu versetzen und/oder zum anderen Luft des Innenbereiches I des Raumes 1 durch eine Entlüftungsbohrung 17 nach außen, d.h. in Richtung des Außenbereiches A, zu transportieren bzw. zu bewegen.

Die dazu notwendige Entlüftungsbohrung 17 ist vorzugsweise in dem Deckenbereich 2 angeordnet und erstreckt sich folglich durch die gesamte Dicke des Deckenbereiches, d.h., durch die Decke 16 sowie auch durch die Außenwand 11 im Deckenbereich 2, um einen Entlüftungsschacht zwischen dem Innenbereich 1 des Raumes 1 und dem Außenbereich A des Raumes 1 bzw. der Außenumgebung A auszubilden.

Der Bodenbereich 4 weist eine erste Holzplatte 18 sowie eine Bodenplatte 19 auf, die beabstandet voneinander, übereinander angeordnet sind, so dass sich zwischen der Holzplatte 18 und der Bodenplatte 19 ein Hohlraum 20 bildet, welcher vorzugsweise mit Bernstein 8 gefüllt ist.

Die Bodenplatte 19 weist eine Vielzahl von Luftlöchern 21 auf, welche sich durch die gesamte Dicke der Bodenplatte erstrecken, wobei deren Durchmesser, sowie deren Anordnung zueinander, d.h. deren Beabstandung zueinander, je nach Anforderung des Raumes bzw. nach gewünschter Erfordernis unterschiedlich sein kann.

Zwischen dem Bernstein 8 im Bodenbereich können auch weitere Lichtleiter (hier nicht gezeigt) angeordnet sein, welche sich durch alle oder nur einen Teil der Luftlöcher 21 erstrecken, um den über den Luftlöchern 21 angeordneten Bernstein 8 zu beleuchten.

Die Ausführungsform des erfindungsgemäßen Raumes 1 gemäß der Figur 1 weist Im Innenbereich I des Raumes 1 an den Wandbereichen 3, dem Deckenbereich 2 sowie im Bodenbereich 4 Bernstein 8 auf, so dass der gesamte Raum1 folglich vollständig mit Bernstein 8 ausgekleidet ist. Der Bernstein 8 selbst kann dabei die unterschiedlichste Form und Größe aufweisen.

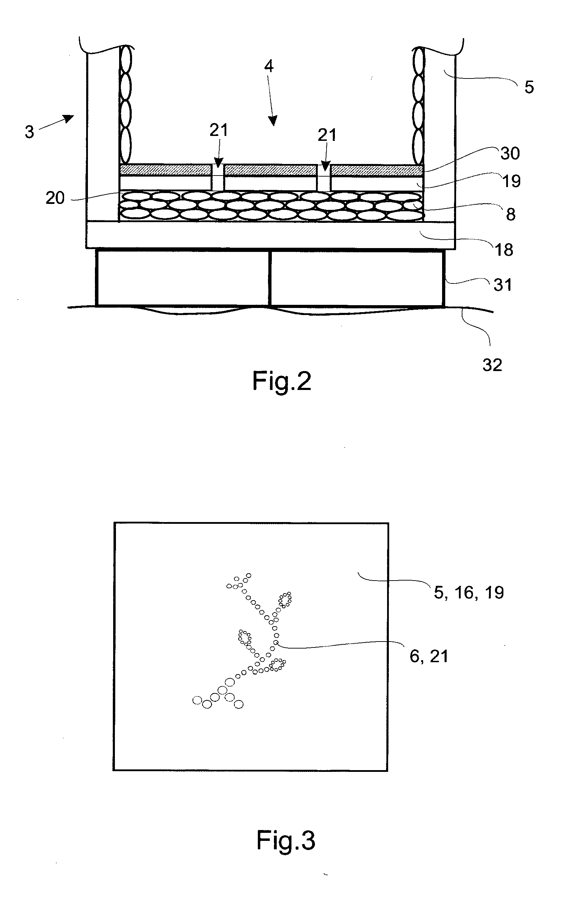

In der Fig.2 ist eine Prinzipskizze eines Längsschnittes durch einen Bodenbereich 4 einer Ausführungsform des erfindungsgemäßen Raumes 1 (siehe Fig.1) dargestellt, welcher sich von dem in Fig.1 dargestellten Bodenbereich 4 unterscheidet, da hier anstatt des auf der Bodenplatte 19 im Innenbereich I des Raumes angeordneten Bernsteins 8 ein Bodenbelag 30, wie beispielsweise ein Parkettboden aufgebracht ist.

Dieser Bodenbelag 30 weist die gleichen Anzahl und Ausbildung von Luftlöchern 21 auf, wie die Bodenplatte 19, so dass sich folglich die Luftlöcher 21 durch die Dicke der Bodenplatte genauso wie durch die Dicke des Bodenbelages erstrecken, um dem in einem Hohlraum 20, welcher sich zwischen der Bodenplatte 19 und der ersten Holzplatte 18 bildet, angeordneten Bernstein 8 zu belüften.

Der Bodenbelag 30 weist vorzugsweise eine Dicke von 21 mm auf, kann jedoch auch dünner oder dicker ausgeführt sein und könnte sich beispielsweise aus Birke-Multiplex-Platten zusammensetzen.

Der gesamte Bodenbereich 4 ist vorzugsweise auf einem Gerüst 31, welches auf dem Erdboden 32 steht, angeordnet, so dass der Bodenbereich 4 zum Erdboden 32 der Außenumgebung A beabstandet angeordnet ist.

Dieses Gerüst 31 ist beispielsweise ein Holzgerüst oder ein aus einem anderen Material, wie Beton hergestelltes Gerüst.

Die Fig.3 zeigt eine Prinzipskizze eines Boden- 4 und/oder Wand- 3 und oder Deckenbereiches 3 bzw. einer Decke 16, einer zweiten Holzplatte 5 und/oder einer Bodenplatte 19 mit ein Symbol bildenden Bohrungen 6 und bzw. Luftlöchern 21.

Hierbei ist deutlich zu erkennen, dass die Bohrungen 6 bzw. Luftlöcher 21 unterschiedliche Durchmesser aufweisen können und derart aneinander angeordnet sind, dass die Gesamtheit der Bohrungen 6 bzw. Luftlöcher 21, durch welche vorzugsweise ein Lichtleiter (hier nicht gezeigt) hindurch gesteckt ist, ein Symbol bzw. ein Bild erzeugt, welche mittels der Lichtleitern (hier nicht gezeigt) erleuchtet bzw. beleuchtet wird.

### Bezugszeichenliste

- 1: Raum
- 2: Deckenbereich
- 3: Wandungsbereich
- 4: Bodenbereich
- 5: zweite Holzplatte
- 6: Bohrung
- 7: Lichtleiter
- 8: Bernstein
- 9: Belüftungsbohrung
- 10: erster Ventilator
- 11: Außenwand
- 12: Hohlraum
- 13: Wärmequelle
- 14: Rauchmelder
- 15: zweiter Ventilator
- 16: Decke
- 17: Entlüftungsbohrung
- 18: erste Holzplatte
- 19: Bodenplatte
- 20: Hohlraum
- 21: Luftloch
- 22: Zerstäuber
- 30: Bodenbelag
- 31: Gerüst
- 32: Erdboden
- A: Außenumgebung
- I: Innenbereich

## Patentansprüche

1. Von einer Außenumgebung (A) abgegrenzter Raum (1) zur Darstellung von Lichtbildern in einem Innenbereich (I) des Raumes (1) mit mindestens einem Wandbereich (3) aus mindestens vier Wänden, einem Bodenbereich (4), einem Deckenbereich (2) und einem verschließbaren Eingangsbereich sowie mit einer Holzkonstruktion (5, 18, 19) zur Stabilisierung mindestens des Wandbereiches (3) und des Bodenbereiches (4) des Raumes (1),
**dadurch gekennzeichnet, dass**
des Raum (1) und des Eingangsbereich ein Wesentlichen schall- und lichtundurchlässig sind; und
die Holzkonstruktion (5, 18, 19) im Bodenbereich (4) mindestens eine erste Holzplatte (18) und eine darüber beabstandet angeordnete Bodenplatte (19) aufweist, wobei zwischen der Bodenplatte (19) und der ersten Holzplatte (18) eine Vielzahl von Bernsteinen (8) angeordnet sind.

2. Raum (1) gemäß Anspruch 1,
**dadurch gekennzeichnet, dass**
die Holzkonstruktion (5, 18, 19) im Wandbereich (3) mindestens eine zweite Holzplatte (5) umfasst, welche durch eine Vielzahl von in der zweiten Holzplatte (5) angeordneten Bohrungen (6) gebildete Symbole aufweist.

3. Raum (1) gemäß Anspruch 2,
**dadurch gekennzeichnet, dass**
durch die Bohrungen (6) Lichtleiter (7) in den Innenbereich (I) des Raumes (1) geführt sind, welche vor den Bohrungen (6) angeordnete und im Innenbereich (I) des Raumes (1) befindliche Bernsteine (8) beleuchten.

4. Raum (1) gemäß Anspruch 3,
**dadurch gekennzeichnet, dass**
die Lichtleiter (7) Glasfasern mit unterschiedlich einstellbarer Farbgebung sind.

5. Raum (1) gemäß Anspruch einem der vorangegangenen Ansprüche,
**dadurch gekennzeichnet, dass**
die Bodenplatte (19) eine Vielzahl von Luftlöchern (21) aufweist, um eine Belüftung des Bernsteines (8) zu ermöglichen.

6. Raum (1) gemäß Anspruch 5,
**dadurch gekennzeichnet, dass**
die Luftlöcher (21) einen Durchmesser von 1 - 20mm und besonders bevorzugt von 5 - 10mm aufweisen.

7. Raum (1) gemäß Anspruch 5 oder 6,
**dadurch gekennzeichnet, dass**
die Luftlöcher (21) über die Bodenplatte (19) verteilt voneinander beabstandet sind und der Abstand der Luftlöcher (21) zueinander vorzugsweise 40 - 80mm und besonders bevorzugt 50 - 60mm beträgt.

8. Raum (1) gemäß einem der Ansprüche 2 bis 4,
**dadurch gekennzeichnet, dass**
die zweite Holzplatte (5) im Wandbereich (3) mindestens eine Belüftungsbohrung (9) aufweist, welche Luft der Außenumgebung (A) in den Innenbereich (I) des Raumes (1) leitet.

9. Raum (1) gemäß Anspruch 8,
**dadurch gekennzeichnet, dass**
innerhalb der Belüftungsbohrung (9) mindestens ein erster Ventilator (10) angeordnet ist, welcher Luft von der Außenumgebung (A) in den Innenbereich (I) des Raumes (1) fördert.

10. Raum (1) gemäß einem der vorangegangenen Ansprüche,
**dadurch gekennzeichnet, dass**
eine Entlüftungsbohrung (17) in dem Deckenbereich (2) des Raumes (1) angeordnet ist, um Luft aus dem Innenbereich (I) des Raumes (1) nach außen zu leiten.

11. Raum (1) gemäß Anspruch 10,
**dadurch gekennzeichnet, dass**
im Innenbereich (I) des Raumes (1) im Wesentlichen unterhalb der im Deckenbereich (2) angeordneten Entlüftungsbohrung (17) ein zweiter Ventilator (15) angeordnet ist, welcher Luft im Innenbereich (I) des Raumes (1) umwälzt und diese Luft von dem Innenbereich (I) des Raumes (1) nach außen fördert.

12. Raum (1) gemäß einem der vorangegangenen Ansprüche,
**dadurch gekennzeichnet, dass**
an dem Deckenbereich (2) des Raumes (1) eine Wärmequelle angeordnet ist, um den Innenbereich (I) des Raumes (1) zu erwärmen und/oder ein Zerstäuber (22), um Bernsteinstaub in dem Raum (1) zu zerstäuben.

## Claims

1. A room (1) bounded off from an external environment (A) for the display of photographs in an inner area (I) of the room (1), with at least one wall area (3) comprising at least four walls, a base area (4), a ceiling area (2) and a closable entry area and with a wooden structure (5, 18, 19) for the stabilization of at least the wall area (3) and the base area (4) of the room (1), **characterized in that** the room (1) and the entry area are substantially impervious to sound and light, and in the base area (4) the wooden structure (5, 18, 19) has at least one first wooden plate (18) and a base plate (19) arranged at a distance above it, wherein a plurality of pieces of amber (8) are arranged between the base plate (19) and the first wooden plate (18).

2. A room (1) according to claim 1, **characterized in that** in the wall area (3) the wooden structure (5, 18, 19) comprises at least one second wooden plate (5), which has symbols formed by a plurality of bores (6) arranged in the second wooden plate (5).

3. A room (1) according to claim 2, **characterized in that** optical conductors (7), which illuminate pieces of amber (8) arranged in front of the bores (6) and situated in the inner area (I) of the room (1), are guided through the bores (6) into the inner area (I) of the room (1).

4. A room (1) according to claim 3, **characterized in that** the optical conductors (7) are glass fibres with colouring capable of being set in different ways.

5. A room (1) according to any one of the preceding claims, **characterized in that** the base plate (19) has a plurality of vents (21) in order to permit an aeration of the amber (8).

6. A room (1) according to claim 5, **characterized in that** the vents (21) have a diameter of from 1 to 20 mm, and in a particularly preferred manner of from 5 to 10 mm.

7. A room (1) according to claim 5 or 6, **characterized in that** the vents (21) are arranged distributed over the base plate (19) at a distance from one another, and the distance of the vents (21) from one another amounts preferably to from 40 to 80 mm, and in a particularly preferred manner to from 50 to 60 mm.

8. A room (1) according to any one of claims 2 to 4, **characterized in that** in the wall area (3) the second wooden plate (5) has at least one aeration bore (9) which conveys air of the external environment (A) into the inner area (I) of the room (1).

9. A room (1) according to claim 8, **characterized in that** at least one first ventilator (10), which drives air from the external environment (A) into the inner area (I) of the room (1), is arranged inside the aeration bore (9).

10. A room (1) according to any one of the preceding claims, **characterized in that** a ventilation bore (17) is arranged in the ceiling area (2) of the room (1) in order to convey air out of the inner area (I) of the room (1) to the outside.

11. A room (1) according to claim 10, **characterized in that** a second ventilator (15), which circulates air in the inner area (I) of the room (1) and drives this air from the inner area (I) of the room (1) to the outside, is arranged substantially below the ventilation bore (17) arranged in the ceiling area (2) in the inner area (I) of the room (1).

12. A room (1) according to any one of the preceding claims, **characterized in that** a heat source to heat the inner area (I) of the room (1) and/or an atomizer (22) to atomize amber dust in the room (1) is or are arranged in the ceiling area (2) of the room (1).

## Revendications

1. Pièce (1), délimitée par rapport à l'environnement extérieur (A), pour la représentation de photographies dans un espace intérieur (I) de la pièce (1), comportant au moins une zone de paroi (3) avec au moins quatre parois, une zone de plancher (4), une zone de plafond (2) et une zone d'entrée pouvant être fermée, et comportant une construction en bois (5, 18, 19) pour stabiliser au moins la zone de paroi (3) et la zone de plancher (4) de la pièce (1),
**caractérisée en ce que**
la pièce (1) et la zone d'entrée sont sensiblement isolées contre le bruit et ne laissent pas passer la lumière ; et
la construction en bois (5, 18, 19) dans la zone de plancher (4) comporte au moins un premier panneau de bois (18) et un panneau de plancher (19) disposé au-dessus et à distance de celui-ci, une pluralité d'ambres jaunes (8) étant disposés entre le panneau de plancher (19) et le premier panneau de bois (18).

2. Pièce (1) selon la revendication 1, **caractérisée en ce que**
la construction en bois (5, 18, 19), dans la zone de paroi (3), comporte au moins un deuxième panneau de bois (5) qui comporte des symboles formés par une pluralité de forures (6) disposées dans le deuxième panneau de bois (5).

3. Pièce (1) selon la revendication 2, **caractérisée en ce qu'**
à travers les forures (6) sont guidés des conducteurs optiques (7) dans l'espace intérieur (I) de la pièce (1) qui éclairent les ambres jaunes (8), disposés en amont des forures (6) et situés dans l'espace intérieur (I) de la pièce (1).

4. Pièce (1) selon la revendication 3, **caractérisée en ce que**
les conducteurs optiques (7) sont des fibres de verre avec une coloration différemment réglable.

5. Pièce (1) selon l'une quelconque des revendications précédentes, **caractérisée en ce que**
le panneau de plancher (19) comporte une pluralité de trous d'air (21) pour permettre une ventilation des ambres jaunes (8).

6. Pièce (1) selon la revendication 5, **caractérisée en ce que**
les trous d'air (21) ont un diamètre de 1 à 20 mm et, de manière particulièrement préférée, de 5 à 10 mm.

7. Pièce (1) selon la revendication 5 ou 6, **caractérisée en ce que**
les trous d'air (21) sont répartis à distances les uns des autres sur le panneau de plancher (19) et la distance entre les trous d'air (21) mesure de préférence 40 à 80 mm et, de manière particulièrement préférée, 50 à 60 mm.

8. Pièce (1) selon l'une quelconque des revendications 2 à 4, **caractérisée en ce que**
le deuxième panneau de bois (5), dans la zone de paroi (3), comporte au moins une forure d'aération (9) qui guide l'air de l'environnement extérieur (A) vers l'espace intérieur (I) de la pièce (1).

9. Pièce (1) selon la revendication 8, **caractérisée en ce que**
à l'intérieur de la forure d'aération (9) est disposé au moins un premier ventilateur (10), qui transporte l'air de l'environnement extérieur (A) vers l'espace intérieur (I) de la pièce (1).

10. Pièce (1) selon l'une quelconque des revendications précédentes, **caractérisée en ce que**
une forure de sortie d'air (17) est disposée dans la zone de plafond (2) de la pièce (1) afin de guider vers l'extérieur l'air provenant de l'espace intérieur (I) de la pièce (1).

11. Pièce (1) selon la revendication 10, **caractérisée en ce que**
dans l'espace intérieur (I) de la pièce (1), sensiblement au-dessous de la forure de sortie d'air (17), agencée dans la zone de plafond (2), est disposé un deuxième ventilateur (15), qui brasse l'air dans l'espace intérieur (I) de la pièce (1) et transporte cet air vers l'extérieur à partir de l'espace intérieur (I) de la pièce (1).

12. Pièce (1) selon l'une quelconque des revendications précédentes, **caractérisée en ce qu'**
au niveau de la zone de plafond (2) de la pièce (1) est disposée une source de chaleur pour chauffer l'espace intérieur (I) de la pièce (1) et/ou un pulvérisateur (22) pour pulvériser de la poussière d'ambre jaune dans la pièce (1).
